**Europäisches Patentamt**

(19) **European Patent Office**

**Office européen des brevets**

(11) Numéro de publication : **0 201 416 B1**

(12) **FASCICULE DE BREVET EUROPEEN**

(45) Date de publication du fascicule du brevet :
10.04.91 Bulletin 91/15

(51) Int. Cl.$^5$ : **C12N 15/43,** C12N 15/33, A61K 39/29

(21) Numéro de dépôt : **86400944.4**

(22) Date de dépôt : **29.04.86**

(54) **Particules ayant les propriétés immunogènes de l'antigène HBs et portant un site antigénique étranger aux épitopes portés par l'antigène HBs, vecteurs et cellules animales pour la production de telles particules et compositions contenant de telles particules pour la production de vaccins mixtes.**

(30) Priorité : **02.05.85 FR 8506708**

(43) Date de publication de la demande :
**12.11.86 Bulletin 86/46**

(45) Mention de la délivrance du brevet :
**10.04.91 Bulletin 91/15**

(84) Etats contractants désignés :
**AT BE CH DE FR GB IT LI LU NL SE**

(56) Documents cités :
**EP-A- 0 119 342
EP-A- 0 180 490
FR-A- 2 532 850
FR-A- 2 550 203
US-A- 4 415 491**

(73) Titulaire : **INSTITUT PASTEUR
25/28, rue du Docteur Roux
F-75015 Paris (FR)**

(72) Inventeur : **Delpeyroux, Francis
89, rue Blomet
F-75015 Paris (FR)**
Inventeur : **Chenciner, Nicole
11, quai Bourbon
F-75004 Paris (FR)**
Inventeur : **Lim, Annick
4, ruedu Castel
F-94000 Creteil (FR)**
Inventeur : **Malpiece, Yves
320, rue St. Fuscien
F-80000 Amiens (FR)**
Inventeur : **Streeck, Rolf
17bis avenue Foch
F-75016 Paris (FR)**

(74) Mandataire : **Gutmann, Ernest et al
S.C. Ernest Gutmann - Yves Plasseraud 67,
boulevard Haussmann
F-75008 Paris (FR)**

EP 0 201 416 B1

Jouve, 18, rue Saint-Denis, 75001 PARIS

## Description

L'invention concerne des particules polypeptidiques le plus souvent sensiblement sphériques, au moins en ce qui concerne la plupart d'entre elles, ces particules ayant les propriétés immunogéniques et immunologiques caractéristiques de l'antigène de surface (souvent désigné par l'abréviation HBsAg ou encore plus simplement HBs) du virus de l'hépatite virale B et portant en outre au moins une séquence peptidique étrangère au polypeptide normalement codé par le gène S du virus de l'hépatite B. L'invention concerne également des ADNs recombinants et des lignées cellulaires eucaryotes ; de préférence d'origine animale, capables d'excréter dans leur milieu de culture des particules polypeptidiques du genre sus-indiqué.

On rappellera tout d'abord que le sérum des porteurs chroniques du virus de l'hépatite B (HBV) contient des enveloppes virales vides sous formes de particules ou filaments de 22 nm de diamètre et parfois des virions complets infectieux, particules sphériques de 42 nm.

Les enveloppes vides, une fois purifiées à partir de sérum de porteurs chroniques du virus, sont utilisées pour la fabrication de vaccins contre l'hépatite B. On sait qu'il est maintenant également possible d'obtenir des particules de 22 nm en grande quantité, par d'autres procédés. Les manipulations génétiques du gène codant pour la protéine majeure des particules (Gène S) ont permis leur production dans des lignées cellulaires en culture (M.F. DUBOIS et al., (1980), Proc. Natl. Acad. Sci. USA, 77, 4549-4553), dans des levures (P. VALENZUELA et al., (1982), Nature, 298, 347-350) ou par l'intermédiaire de virus recombinants (G.L. SMITH et al. (1983), Nature, 302, 490-495). Une méthodologie pour produire ces particules comprend la transformation de cellules eucaryotes par un vecteur approprié, contenant le gène S sous la dépendance d'un promoteur efficace, la culture des cellules transformées et la récupération des particules produites, soit à partir des cellules préalablement lysées, soit à partir du milieu de culture, lorsque les particules y ont été excrétées par les lignées cellulaires utilisées (notamment dans le cas de l'utilisation de cellules de singe, par exemple du type VERO).

Le polypeptide majeur codé par le gène S, entrant dans la constitution de ces particules, est constitué de 226 acides aminés et possède un poids moléculaire de 25.400 daltons. Il a été montré également que dans le polypeptide constitutif, certaines particules naturelles pouvaient également être constituées d'un polypeptide de poids moléculaire plus élevé, de l'ordre de 34.000 daltons, contenant la séquence polypeptidique du susdit polypeptide majeur, ayant la même extrémité C-terminale que le polypeptide majeur et en outre une séquence supplémentaire de 55 acides aminés en position N-terminale (STIBBE X. et GERLICH W.H., (1983), J. Virology, 46, 626-628) codée par la région pré-S du génome de l'hépatite B. Cette séquence supplémentaire en position N-terminale n'est apparemment pas très stable dans les particules naturelles, et ne semble donc pas jouer un rôle important dans la constitution et la cohésion des particules d'HBs. Celles-ci, on le sait, sont constituées en aggrégats organisés, peu sensibles aux protéases, et impliquant une centaine desdits polypeptides majeurs et d'autres constituants, plus particulièrement lipidiques. Un procédé permettant l'obtention de compositions contenant une proportion notable, pouvant atteindre 35% du total des polypeptides formés, de particules plus stables contenant ladite séquence supplémentaire a récemment été décrit (MICHEL, M.L. et al., (1984), Proc. Natl. Acad. Sci. USA, 81, 7708-7712). Il met en oeuvre des lignées cellulaires eucaryotes, plus particulièrement de cellules humaines ou animales en culture, transformées au préalable par des vecteurs, contenant une séquence d'ADN codant pour les régions S et pré-S du génome du virus de l'hépatite virale B, placée à l'intérieur de ce vecteur, sous le contrôle direct d'un promoteur exogène dont est connue la capacité de permettre l'initiation efficace de la transcription des gènes directement sous son contrôle dans les cellules eucaryotes, notamment humaines ou animales, auxquelles lesdits vecteurs sont destinés. On se reportera par exemple à l'article de GALIBERT et Col., (1979), Nature, vol. 281, p. 646-650, pour ce qui est de ladite séquence d'ADN.

Lorsque les lignées cellulaires utilisées sont originaires du singe, il est avantageux d'avoir recours à un promoteur issu du virus SV40, dont est connue la capacité de permettre l'initiation efficace de la transcription de gènes adjacents dans des cellules de singe. Avantageusement, ce promoteur correspond au promoteur "précoce" 'du virus SV40, lequel contrôle normalement l'expression de l'antigène "petit T" ("small T antigen") et également de l'antigène "grand T" ("large T antigen").

La variabilité naturelle de l'extrémité N-terminale des polypeptides de l'enveloppe du virus de l'hépatite B a déjà donné à penser que des fragments de protéines distincts de la susdite séquence supplémentaire pouvaient lui être substitués et fusionnés avec le sus-dit polypeptide majeur. C'est ce qu'ont réalisé VALENZUELA et al., qui ont obtenu dans des levures transformées des particules formées à partir de protéines hybrides consistant essentiellement en le susdit polypeptide majeur modifié à son extrémité N-terminale par un polypeptide supplémentaire comportant une centaine d'acides aminés issus de la glycoprotéine D du virus de l'Herpès. P. VALENZUELA et al. rapportent que ces particules transformées étaient capables d'induire des anticorps contre à la fois le virus de l'hépatite B et le virus de l'Herpès (P. VALENZUELA et al (1982), Nature, 298, 347-350 et P. VALENZUELA (1984) "In Proceedings of the Twelwth International Conference on Yeast Genetics and Molecular Biology" (Travaux de la douzième Conférence Internationale sur la Génétique des levures et de la biologie moléculaire), Edimbourg, (1984), 16).

La présente invention a pour but de fournir des particules polypeptidiques, ayant les propriétés immunogènes de base de l'antigène HBs et contenant en outre au moins une autre séquence peptidique, de préférence également immunogène, en d'autres termes des particules polypeptidiques susceptibles d'être utilisées pour la constitution de vaccins mixtes, ayant une stabilité optimum, l'autre séquence peptidique devant être présente chaque fois que le polypeptide majeur, lui-même ou de préférence la glycoprotéine majeure elle-même de l'antigène HBs, se trouve être synthétisé, et ce sans que soit sensiblement affectée l'architecture particulaire caractéristique des antigènes d'enveloppe du virus de l'hépatite B.

Elle a encore pour but l'obtention de particules de ce type qui peuvent, le cas échéant, contenir en outre la séquence supplémentaire normalement codée par la région pré-S du génome du virus de l'hépatite B, à l'état intact, étant cependant entendu que celle-ci pourrait aussi être modifiée, par exemple selon les modalités envisagées par VALENZUELA et al. Mais la préservation du caractère intact de ladite séquence supplémentaire réside dans l'immunogénicité accrue qui peut en résulter pour les polypeptides modifiés conformes à l'invention.

Les particules selon l'invention qui contiennent une proportion suffisante des séquences d'aminoacides caractéristiques du polypeptide majeur de l'antigène HBs, pour conserver à ces particules la structure caractéristique de l'antigène HBs, sont caractérisées par l'incorporation à ce polypeptide majeur d'au moins une séquence d'aminoacides étrangère à ce polypeptide majeur, de préférence elles-mêmes porteuses d'un site immunogène, à l'intérieur même de ce polypeptide majeur, notamment en l'une de ses régions hydrophiles normalement exposées à la surface extérieure desdites particules ou, en variante, par la substitution de un ou plusieurs aminocides appartenant à ces régions hydrophiles par ladite séquence étrangère d'aminoacides.

En particulier la séquence étrangère d'aminoacides est insérable dans l'une des régions s'étendant entre les acides aminés 32 à 74 ou entre les acides aminés 110 à 156 du polypeptide majeur dont des formules générales ont été présentées dans l'article de P. TIOLLAIS et al. (1981) SCIENCE, vol. 213, pp. 406-411, formules qui sont rappelées ci-après :

A

```
  1   Met Glu Asn Ile Thr Ser Gly Phe Leu Gly Pro Leu Leu Val Leu Gln Ala Gly Phe Phe

 21   Leu Leu Thr Arg Ile Leu Thr Ile Pro Gln Ser Leu Asp Ser Trp Trp Thr Ser Leu Asn

                      Ser Pro                                             Thr
 41   Phe Leu Gly Gly Thr Thr Val Cys Leu Gly Gln Asn Ser Gln Ser Pro Thr Ser |Asn His
                      Thr Thr                                             Ile

                                  Ile
 61   |Ser Pro Thr Ser Cys Pro Pro Thr Cys Pro Gly Tyr Arg Trp Met Cys Leu Arg Arg Phe
                                  Thr

 81   Ile Ile Phe Leu Phe Ile Leu Leu Leu Cys Leu Ile Phe Leu Leu Val Leu Leu Asp Tyr

                                                      Thr                           Pro
101   Gln Gly Met Leu Pro Val Cys Pro Leu Ile Pro Gly Ser Ser Thr Thr Ser Thr Gly Pro
                                                      Ser                           Ser

              Lys         Thr         Pro             Asn     Phe
121   Cys Arg Thr Cys Met Thr Thr Ala Gln Gly Thr Ser Met Tyr Pro Ser Cys Cys Cys Thr
              Arg         Thr         Pro             Ile     Tyr

          Thr                                                                 Ala
141   Lys Pro Ser Asp Gly |Asn Cys Thr| Cys Ile Pro Ile Pro Ser Ser Trp Ala Phe Gly Lys
              Ser                                                             Gly

      Tyr                       Val
161   Phe Leu Trp Glu Trp Ala Ser Ala Arg Phe Ser Trp Leu Ser Leu Leu Val Pro Phe Val
      Phe                       Ala

                                      Thr                       Ala
181   Gln Trp Phe Val Gly Leu Ser Pro Thr Val Trp Leu Ser Val Ile Trp Met Met Trp Tyr
                                      Ile                       Val
                                      Val                       Ile
201   Trp Gly Pro Ser Leu Tyr Ser Ile Leu Ser Pro Phe Leu Pro Leu Leu Pro Ile Phe Phe
                          Val           Leu             Leu

221   Cys Leu Trp Val Tyr Ile
                      Ala
```

Il est rappelé que la formule du peptide majeur est sujette à variations. En particulier les différences au niveau des acides aminés constitutifs observés par VALENZUELA et al. (1980) "Animal Virus Genetics" (Génétique des virus d'animaux), B. FIELDS, R. JAENISCH, C.F. FOX, Ed : Academic Press, New York, p. 57, apparaissent au-dessus des lignes principales de ladite formule, celles observées par Pasek et al, Nature (London), 282, 575 (1979), apparaissent au-dessous desdites lignes principales.

L'invention concerne donç plus particulièrement des compositions utiles pour la fabrication de vaccins qui contiennent des particules polypeptidiques sensiblement sphériques (ou qui est formée par ces particules), au moins en ce qui concerne la plupart d'entre elles (sinon toutes), qui ont les propriétés immunogéniques et immunologiques caractéristiques de l'antigène HBsAg, qui ont des tailles de 18 à 25 nm, notamment de 20 à 22 nm, et des densités permettant leur isolement dans une zone de densité de 1,20-1,22 g/ml dans un gradient de densité à base de CsCl, et un niveau de pureté totale pour ce qui est de l'absence de toute particule de Dane et d'antigène HBe, y inclus HBc, ces particules étant plus particulièrement caractérisées par la présence desdites séquences étrangères dans les conditions sus-indiquées.

La taille des séquences étrangères susceptibles d'être insérées à l'intérieur du polypeptide majeur, tel qu'il vient d'être défini, peut être modifiée dans de grandes proportions. Il est possible d'introduire des séquences d'aminoacides pouvant comporter par exemple jusqu'à 100 aminoacides, voire davantage. Il est cependant avantageux que la séquence peptidique étrangère ait une taille ne dépassant pas 16 aminoacides, notamment de 5 à 16, par exemple de 6 à 13, surtout dans le cas où elle est insérée dans le polypeptide majeur, sans suppression d'un nombre sensiblement équivalent des aminoacides que celui-ci pouvait comprendre auparavant. En effet, l'invention permet le résultat remarquable que constitue la possibilité de produire dans ceux des systèmes cellulaires qui le permettent, l'excrétion des particules modifiées conformes à l'invention par les cellules concernées, lorsque celles-ci ont été transformées au préalable par un vecteur approprié contenant une séquence d'ADN codant pour le polypeptide modifié conforme à l'invention.

L'invention concerne naturellement également les ADNs recombinants codant pour lesdits polypeptides modifiés entrant dans la composition des particules sus-indiquées. A cet égard, ces ADNs recombinants, et de préférence des vecteurs les contenant, qui contiennent une séquence d'ADN codant pour la région S et, le cas échéant, pré-S du génome du virus de l'hépatite virale B, sont caractérisés en ce que ladite séquence d'ADN est localement modifiée par au moins une séquence nucléotidique codant pour la susdite séquence étrangère, en une au moins de celles des zones de la région S correspondant aux régions hydrophiles du polypeptide majeur susdit, et en ce que la région S et, le cas échéant, la région pré-S, sont à l'intérieur de l'ADN recombinant, placé sous le contrôle direct d'un promoteur exogène dont est connue la capacité de permettre l'initiation efficace de la transcription des gènes directement sous son contrôle dans les cellules eucaryotes, notamment humaines ou animales, ou encore aux levures auxquelles lesdits vecteurs sont destinés.

Le promoteur exogène mis en oeuvre est distinct ou étranger vis-à-vis du promoteur "endogène", normalement associé aux gènes S et pré-S dans le génome du virus de l'hépatite B. Lorsque ces cellules sont originaires du singe, il est avantageux d'avoir recours à l'un des promoteurs issus du virus SV40, qui ont été rappelés plus haut.

L'invention ne se limite cependant pas à l'utilisation de ce promoteur particulier, bien que celui-ci donne des résultats particulièrement favorables, eu égard à la production par les cellules transformées de polypeptides hybrides conformes à l'invention, caractéristiques de l'antigène HBs et d'un récepteur de la pHSA, et à leur excrétion dans le milieu de culture utilisé. On peut également avoir recours par exemple au promoteur tardif de SV40 (qui contrôle l'expression des protéines VP1, VP2 et VP3). On peut se reporter à la carte de restriction du virus SV40 (J. TOOZE, Ed. DNA Tumor Viruses, Cold Spring Harbor Laboratory, Cold Spring Harbor, N.Y., 1980, chaps. 2-5), pour apprécier les positions relatives de ces promoteurs et des gènes codant pour les différents antigènes qui leur sont associés

Il va de soi que l'on peut substituer aux promoteurs de SV40 tout autre type de promoteur connu comme possédant ou dont pourrait être découverte la capacité de promouvoir la transcription dans les lignées cellulaires mises en oeuvre desdites séquences codant pour les susdites régions S et, le cas échéant, pré-S, dès lors qu'elles seraient placées sous leur contrôle, avec pour résultat l'incorporation de ces séquences avec ce promoteur dans le génome des cellules réceptrices et/ou la capacité conférée aux cellules réceptrices ainsi transformées de synthétiser et d'excréter des quantités substantielles du polypeptide hybride selon l'invention, la capacité ainsi acquise étant ensuite transmissible aux générations successives issues de ces cellules.

Les dites lignées transformées seront dites "stables" lorsque le caractère acquis par les lignées cellulaires selon l'invention de synthétiser les susdits polypeptides se transmet d'une génération de cellules à l'autre, sur au moins 10 générations.

A titre d'autres promoteurs susceptibles d'être utilisés, on mentionnera par exemple le promoteur précoce du polyome ou des promoteurs LTR de différents rétrovirus ou encore le promoteur EA de l'adénovirus, ainsi que de promoteurs efficaces de gènes d'origine cellulaire.

Comme il est bien connu, les promoteurs prélevés sur les génomes des virus dont ils sont originaires sont de préférence accompagnés des "séquences" activatrices qui normalement les précèdent (par rapport au sens de la transcription des séquences géniques normalement placées sous leur contrôle). A titre d'exemple de séquences activatrices, on peut se référer à l'article de Science, 1983, vol. 219, pages 626 à 631, et Nature, 1982, vol. 295, pages 568 à 572.

Avantageusement, la susdite séquence d'ADN codant pour les susdites régions pré-S et S est placée, immédiatement derrière un fragment d'ADN constitué par le promoteur et la séquence activatrice permettant la transcription normale de la séquence pré-S ou S. Le susdit fragment comprend notamment de 300 à 400 paires de bases selon le type de promoteur et de séquences activatrices utilisées.

L'invention concerne encore les lignées cellulaires transformées par des vecteurs tels qu'ils ont été définis ci-dessus et qui sont aptes à excréter dans leur milieu de culture les particules immunogènes également définies ci-dessus.

Des lignées préférées selon l'invention sont formées de cellules de mammifères, notamment de cellules CHO ou VERO

L'invention concerne encore un procédé de production de telles lignées cellulaires susceptibles d'être maintenues en culture, ce procédé comportant la transformation de ces lignées avec un vecteur tel que défini ci-dessus et l'isolement de celles des cultures qui expriment les séquences codant pour la protéine hybride de l'invention. Des caractéristiques supplémentaires de l'invention apparaîtront encore au cours de la description qui suit d'exemples de constructions qui illustrent le principe de base de l'invention. Il sera dans ce qui suit fait référence au dessin dans lequel :

– la figure 1 représente la structure schématique du plasmide PLAS utilisé dans les constructions selon l'invention,

– la figure 2 représente la structure schématique du plasmide pPAP dérivé du pLAS et comportant en plus de ce dernier un fragment d'ADN synthétique codant pour une séquence d'onze animo-acides de la protéine VP1 du poliovirus type I (souche Mahoney).

– Construction des plasmides transfectés :

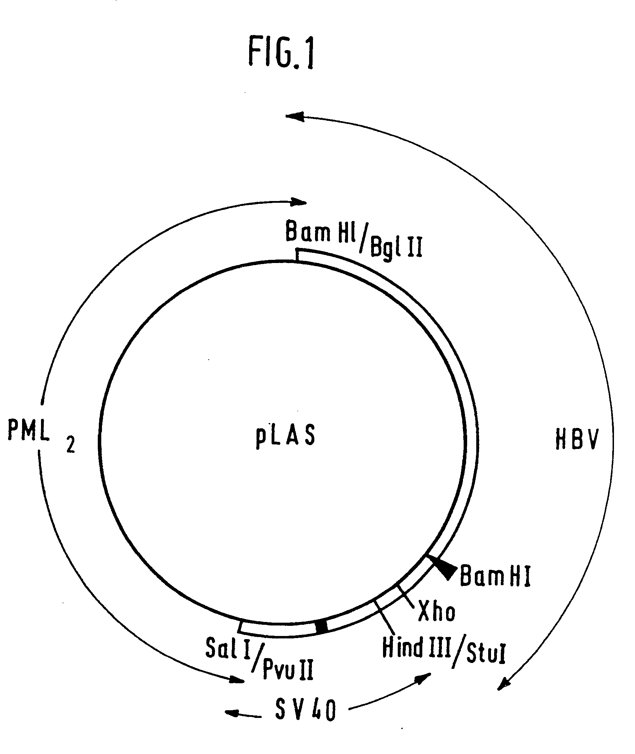

Le plasmide pLAS – Ce plasmide comprend (figure 1) :

– la partie codante du gène S (P. Charnay et al. 1979) avec son site naturel de polyadénylation (fragment Stul (43) Bglll (1984)) dont le site BamHI (1400) est supprimé par réparation par l'enzyme de Klenow ;
– ce gène sans promoteur est mis sous la dépendance du promoteur précoce du virus SV40 (fragment du virus SV40 compris entre le site Pvull (250) – et le site Hindlll (5154) ;
– le grand fragment BamHI (375)-Sall (650) du pML2 (Lusky, M. and Botchan, M. 1981).

Le fragment du gène S a été ligaturé, au niveau de son extrémité Bglll à l'extrémité BamHI du plasmide pML2 (les extrémités Bglll et BamHI étant mutuellement compatibles). Le fragment de gène S a par contre été modifié au niveau de son extrémité Stul par une séquence de liaison nucléotidique (linker) obtenue par synthèse chimique et contenant un site Hind III, pour réaliser une ligation avec l'extrémité Hind III, du fragment de virus SV40 contenant le promoteur susdit. Enfin, l'extrémité Sall du fragment issu du plasmide pML2 avait été réparée avant sa ligation avec l'extrémité Pvull (extrémité franche) du fragment de virus SV40.

Les plasmides pLAS$_I$, pLAS$_{II}$.

Ces deux plasmides sont dérivés du plasmide pLAS décrit ci-dessus par introduction au site unique BamHI (488) d'un ou deux fragments BamH1, d'ADN de 24 paires de bases dérivés du pSKS104 (Shapiro et al. 1983). Ce fragment de 24 nucléotides codant pour 8 acides aminés dont l'insertion dans le gène S ne modifie pas la phase de lecture, porte un site de coupure pour les enzymes de restriction Pstl, et deux sites pour les enzymes Hind II (Sall, Accl).

EXEMPLE I.

Obtention des lignées cellulaires de souris produisant la protéine HBs après transfection puis sélection des clones producteurs.

Des cellules LMTK⁻ (clone 1D ; cellules de souris dérivées du clone L929 déficientes en thymidine kinase, poussant dans le milieu Eagle modifié par Dulbecco (milieu DMEM), supplémenté par 10% de sérum de veau et de glutamine 4 mM) ont été cotransfectées (techniques de Graham et Van der Eb, 1973, modifiées par Wigler et al, 1979) par de l'ADN d'un des trois vecteurs (pLAS, pLAS$_I$, pLAS$_{II}$) et par l'ADN du plasmide pW portant le gène de l'aminoglycoside-3'-phosphotransférase APH3' résistant à la néomycine (Colbère-Garapin et al. 1981). Les cellules transfectées exprimant l'enzyme APH3' sont sélectionnées en présence de 400 microgrammes/millilitre d'aminoglycoside G418 (Colbère-Garapin et al. 1981). Les clones issus de cette sélection sont alors testés pour la production de la protéine HBs. Ainsi $5.10^5$ cellules LMTK⁻ ont été cotransfectées par 10 microgrammes d'ADN du premier plasmide et par 2 microgrammes d'ADN du plasmide pW. Quatre jours après la transfection, le milieu G418 sélectif était appliqué.

Les clones viables apparaissant sont isolés, mis en culture et testés quant à leur capacité de production de l'antigène HBs dans le milieu.

La présence d'HBsAg est détectée à l'aide des tests radioimmunologiques AUSRIA II (Abbott Laboratories). Parmi les clones cellulaires donnant une réponse positive, trois clones (LAS, LASI, LASII) correspondant aux plasmides pLAS, pLASI, pLASII sont sélectionnés pour être caractérisés.

Caractérisation des particules détectées dans le milieu des clones cellulaires.

Les clones amenés à confluence, le milieu nutritif des cellules est changé et mis à accumuler pendant 48 heures. Le surnageant est ensuite clarifié à 2000 rpm et centrifugé pour sédimenter les particules (Smith et al. 1983). Le culot est repris dans du tampon, déposé sur un gradient de CsCl, centrifugé, collecté et les fractions sont testées en R.I.A. (Moriarty et al. 1981). L'activité HBsAg des protéines modifiées et non modifiées se retrouve concentrée en un seul pic compris entre les densités 1,18 et 1,24 g/cm³, densité similaire aux particules purifiées du sérum (Pillot et al. 1984). Un aliquote est alors déposé sur un gradient de sucrose (Moriarty et al. 1981). Après collecte des fractions, l'activité HBsAg sédimente en un seul pic de coefficient de sédimentation apparemment identique entre les trois types de polypeptides.

Caractérisation des séquences du gène S intégrées dans le génome des clones cellulaires LAS, LASI, LASII.

L'ADN cellulaire des clones LAS, LASI, LASII est préparé par la méthode de Gross-Bellard et al. (1973) et digéré par les enzymes de restriction HindIII et PstI. Après électrophorèse sur gel d'agarose, les ADN sont transférés sur une feuille de nitrocellulose (Southern 1975) qui est hybridée avec une sonde radioactive fabriquée à partir d'un fragment d'ADN contenant le gène S selon la méthode de translation de coupure (Rigby et al. 1977).

Après autoradiographie des répliques sur nitrocellulose, on met en évidence qu'un ou plusieurs gènes ont été intégrés dans les trois clones. De plus, un site PstI existe uniquement au niveau du gène S modifié des clones cellulaires LASI et LASII. Ce site PstI n'existe pas dans la séquence naturelle du gène S utilisé (P. Charnay et al. 1979) mais bien dans les fragments d'ADN étrangers insérés dans les plasmides pLASI et pLASII utilisés.

Immunoprécipitation par un sérum anti-HBsAg des protéines excrétées par les clones LAS, LASI et LASII.

Les clones cellulaires à confluence sont marqués avec de la méthionine [³⁵S] pendant 48 heures et les surnageants immunoprécipités par des anticorps de lapin anti-HBsAg (Behring) puis par de la protéine A Sépharose (Pharmacia) en présence de NP40 0,5%. Après lavage, les immunoprécipités sont déposés sur un gel de polyacrylamide 15% d'après la technique décrite par Laemmli (1970) en présence de marqueurs de poids moléculaires (Pharmacia). Les gels traités et séchés sont alors exposés en autoradiographie.

Deux bandes majeures apparaissent pour chaque surnageant. Leur poids moléculaire est respectivement de 23000 et 27000 pour le clone LAS, 24750 et 28500 pour le clone LASI, 26000 et 29000 pour le clone LASII.

Le plasmide pLAS se révèle efficace pour promouvoir l'expression d'HBsAg dans les cellules L. De plus, le plasmide pLASI porte des sites de restriction supplémentaires dans la région codante du gène S, ce qui peut faciliter l'introduction de séquences nouvelles.

Le fait de pouvoir détecter en R.I.A. des structures semblables aux particules d'HBsAg dans les surnageants cellulaires nous permet de dire que les structures induites par les plasmides pLASI et pLASII conservent, au moins, une antigénicité partiellement similaire à celle des particules de sérum humain. L'insertion des 8 ou 16 acides aminés utilisés n'empêche pas l'excrétion des protéines d'HBsAg modifiées du cytoplasme vers le milieu extérieur des cellules L. Le choix du site BamHI pour effectuer des insertions dans le gène S se révèle judicieux. En effet, il correspond au début de la région hydrophile majeure de la protéine (P. Tiollais et coll. 1981) et respecte le fait que les séquences hydrophobes doivent être au contact de la membrane lipidique des particules. Le domaine intermembranaire d'HBsAg responsable de la structure des particules de 22 nm ne subit vraisemblablement que des transitions conformationnelles mineures.

Les analyses sur gradient de chlorure de césium et de sucrose des surnageants des clones cellulaires ne permettent pas, dans les limites expérimentales employées, de mettre en évidence des différences entre les structures modifiées et non modifiées.

– L'analyse des ADN cellulaires montre que les gènes S intégrés portent toujours les modifications apportées sur les plasmides utilisés.

– Les protéines mises en évidence après immunoprécipitation montrent des différences de poids moléculaires attendues. Cependant, le poids moléculaire mesuré des protéines modifiées paraît supérieur au poids moléculaire réel (le poids moléculaire d'un fragment de 8aa est en effet de 957). D'autre part, les modifications permettent toujours la glycosylation partielle de la protéine HBsAg. Les deux bandes apparaissant en gel de polyacrylamide sont caractéristiques d'un polypeptide glycosylé et non glycosylé. Le résidu le plus susceptible d'être glycosylé, l'Asparagine 146 (Machida et coll. 1983) intervient dans la séquence participant au déterminant antigénique majeur a (Pillot et coll. 1984). La conformation de cette partie de la protéine doit donc être relativement similaire dans les protéines modifiées ou natives.

## EXEMPLE II

### Production de particules portant l'antigène de surface de l'hépatite B modifiées par l'insertion de séquences de la toxine diphtérique.

L'ADN du plasmide pTD134 contenant le gène de la toxine diphtérique (M. Kaczorek et coll., 1983) est coupé par l'enzyme HaeIII, traité par la nucléase BAL31 puis ligué, en présence de T4 DNA ligase avec des adaptateurs BamHI. Après coupure avec l'enzyme BamHI, les fragments sont mis à liguer avec l'ADN du plasmide pSKS105 (Shapiro et coll., 1983) coupé par la même enzyme. Cet ADN est alors utilisé pour transformer une souche d'E. coli. Les colonies sont transférées et lysées sur des feuilles de nitrocellulose. Ces dernières sont mises à hybrider avec une sonde radioactive fabriquée par translation de coupure à partir d'un fragment purifié sur gel d'acrylamide après digestion du plasmide pTD134 par l'endonucléase HaeIII (fragment HaeIII 597-HaeIII 746). Les plasmides des colonies hybridant avec cette sonde ont été partiellement séquencés grâce à la méthode de Maxam et Gilbert (Maxam et coll., 1980). Un d'entre eux, contenant l'insertion d'un fragment BamHI-BamHI codant pour les acides aminés 201-231 du gène de la toxine diphtérique (Kaczorek et coll., 1983) a été sélectionné. Ce fragment a ensuite été réintroduit dans le site BamHI du plasmide pLAS.

Le nouveau plasmide pTAS a été purifié et transfecté dans des cellules L de souris. Après sélection des clones cellulaires résistant au G418, selon la méthode décrite au premier paragraphe de l'exemple I, la présence d'HBsAg est testée dans les surnageants. Sur 20 clones examinés, aucun n'est positif.

Les cellules de 10 clones sont trypsinées, lavées 2 fois au P.B.S. et lysées par 3 cycles de congélation-décongélation dans 250 µl de Tris 10 mM pH 7,4, EDTA 1 mM. Le lysat est clarifié à 2000 rpm et testé. Tous les lysats des clones contenaient de l'HBsAg.

Un clone a été lysé dans les mêmes conditions. Des aliquotes ont été déposés sur des gradients de chlorure de césium ou de sucrose en parallèle avec des particules d'HBsAg purifiées à partir de sérum humain (I.P.P.) et un lysat de clone transfecté par le plasmide pLAS produisant des particules non modifiées. Aucune différence n'a pu être détectée entre les particules du sérum humain et les signaux d'HBsAg des lysats étudiés. Cela tendrait à prouver que, même non excrétées, les protéines modifiées sont, après lyse des cellules, dans une conformation relativement similaire à celles des particules "naturelles". L'insertion au niveau du site BamHI (aa112-113) de l'HBsAg de 32 acides aminés codant pour une partie de la toxine diphtérique ne permet plus l'excrétion de la protéine lorsqu'elle est traduite dans des cellules L. C'est ce que montrent les expériences avec le plasmide pTAS. Cependant, le fait de retrouver la protéine modifiée sous forme de particules non excrétées permet de penser qu'il est possible de modifier l'HBsAg et de l'exprimer dans un système non excréteur (la levure par exemple). La persistance de structures particulaires confère une grande stabilité à la protéine, ce qui facilite sa purification.

## EXEMPLE III.

### Production de particules portant l'antigène de surface de l'hépatite B modifiées par l'insertion de séquences du virus polio.

Les 2 brins d'un fragment d'ADN de 47 paires de bases codant pour 11 acides aminés de la protéine VPI du poliovirus type I, (acides aminés 93 à 103) et pour 2 sites reconnus par l'enzyme BamHI ont été synthétisés par voie chimique grâce à un synthétiseur automatique (Applied Biosystem). Les 2 brins ont été purifiés séparément sur un gel dénaturant de polyacrylamide, puis hybridés. Le fragment a été coupé par l'endonucléase

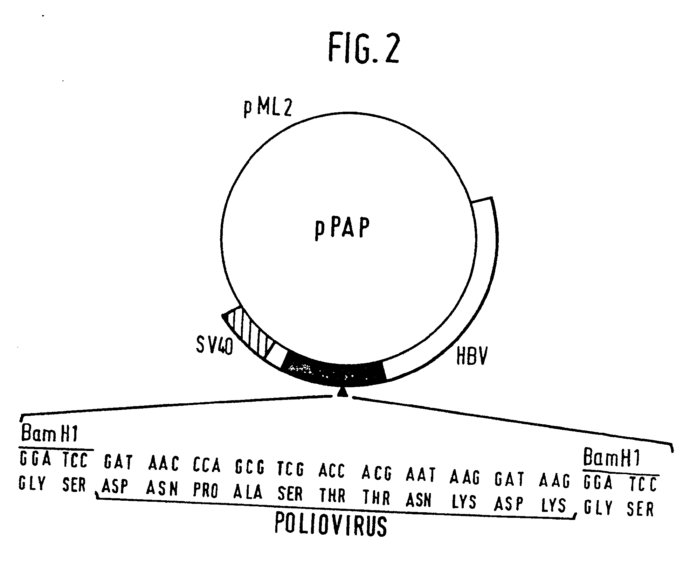

BamHI et inséré au site BamHI du plasmide pLAS. Le nouveau plasmide pPAP (figure 2) a été séquencé partiellement (Maxam et Gilbert, 1980), amplifié et introduit dans des cellules L selon la méthode précedemment décrite dans le premier paragraphe de l'exemple I. Les clones résistant au G418 ont été isolés, leur surnageant testé pour la présence d'HBsAg ; 14 clones sur 20 ont été trouvés positifs.

Pour la purification des particules de 22 nm, les clones cellulaires ont été mis en culture dans le DMEM, et après huit jours, les surnageants cellulaires ont été clarifiés et 45%. d'une solution de sulfate d'ammonium à pH 7,5 ont été additionnés.

Le précipité a été collecté par centrifugation et les granules obtenus ont été dissous dans 10 mM de Tris HCl, pH 7,5, 150 mM de NACl, 1 mM d'EDTA (TNE), et dyalisés contre le même tampon.

Du CsCL, 0,3 mg/ml, a été additionné, suivi d'une centrifugation a 4°C pendant 72 heures a 40 krpm dans un rotor beckman 60 Ti.

Des fractions d'1 ml ont été collectées après centrifugation, et l'HBsAg a été testé par RIA.

Les fractions contenant l'HBsAG ont été regroupées et recentrifugées dans le CsCl à 4°C pendant 48 heures à 47 krpm dans un rotor Beckman 50 Ti.

Des fractions de 0,33 ml ont été récoltées à partir du surnageant et la présence d'HBsAg a été testée encore une fois.

Les fractions correspondantes au pic d'activité HBsAg ont été regroupées et dialysées contre le TNE, et l'HBsAg a été précipité par centrifugation pendant 24 heures à 28 krpm dans un rotor SW41.

Le précipité a été remis en suspension dans 0,5 ml de TNE et placé dans un gradient de saccharose linéaire 10-30% (W/W) dans le TNE avec 0,5 ml de saccharose à 66%.

Après centrifugation pendant 4,5 heures à 35 krpm et 4°C dans un rotor SW41, des fractions de 0,33 ml ont été collectées à partir du surnageant.

Les fractions correspondant au pic d'activité HBsAg ont été regroupées et dialysées contre le TNE. Les particules d'enveloppe purifiées ont été analysées par électrophorèse sur gel SDS-polyacrylamide suivie d'une coloration à l'argent.

La concentration en protéine a été déterminée par la méthode BioRad.

Les particules purifiées à partir de milieux de culture de clones cellulaires (PAP, LAS) transfectées par pPAP ou pLAS respectivement ont environ la même densité dans CsCl. Elles ne diffèrerent pas significativement des particules HBsAg humaines, selon les essais de sédimentation dans la saccharose, mais semblent posséder des diamètres plus variables.

Les polypeptides HBsAg et HBsPolioAg immuno-précipités par un anti-sérum anti-HBsAg obtenu à partir de particules LAS et PAP respectivement, sont présents sous forme gycosylée et non glycosylée.

La différence d'1,5 kDa entre les poids moléculaires apparents de l'HBsAg et de l'HBsPolioAg correspond au poids moléculaire de la séquence insérée.

Les résultats démontrent que l'insertion n'empêche ni les interactions spécifiques entre protéines et lipides nécessaires pour l'assemblage des particules d'enveloppe, ni la glycosylation et la sécrétion des particules par les cellules des milieux de cultures.

Dans le but d'établir si la séquence de poliovirus insérée est bien exposée à la surface des particules et d'examiner si des changements de conformation ont été induits, des études de sensibilité à la protéase des particules HBsAg et HBsPolioAg ont été réalisées.

Des clones cellulaires (LAS, PAP) ont été mis en culture à confluence, lavés deux fois avec du DMEM sans méthionine et ont été incubés pendant deux heures dans le même milieu auquel a été additionné 4 mM de glutamine et 1% de sérum de veau.

L'incubation est prolongée de 24 heures dans un milieu frais contenant $2.10^6$ cellules et 100 µCi/ml de [25]S-Met (100 Ci/mmole ; Amersham).

Après 6 heures d'incubation en présence de 30 µg/ml de Met non marquée, les particules d'enveloppe ont été partiellement purifiées à partir de surnageant du milieu de culture en le centrifugeant pendant 24 heures à 28 krpm et 4°C (rotor SW41), suivi d'une centrifugation à travers un gradient de CsCl ($1,1$-$1,6$ g/cm³)pendant 24 heures a 35 krpm et 4°C.

Des fractions de 0,5 ml ont été collectées et des fractions du pic ont été dialysées contre le pbs. Des aliquots de 100 µl ont été mélangés avec 50 µl de trypsine (300 µg/ml, Worthington) dans du PBS avec ou sans 3% de β-mercaptoethanol et incubées pendant 2 heures à 37°C. Puis addition de 50 µl, d'une solution à 300 µg/ml dans le PBS, d'inhibiteur de la trypsine de soja (Worthington). Le volume a été augmenté de 400 µl avec du PBS et additionné de 1% d'albumine de sérum de boeuf, 1% de desoxycholate de sodium, 0,1% de SDS, et l'immuno-précipitation a été obtenue après une nuit à 4°C en présence d'antisérum de lapin dirigé contre les particules d'HBsAg humaines (Behring) à une dilution d'1 : 100. Puis addition de 50 µl de Sepharose-protéine A remise en suspension dans 1 volume de 25 mM de Tris-HCl pH 7,2, 2,5 mM d'EDTA et 2mM de PMSF

Après une heure d'agitation douce à 4°C, la Sepharose a été lavée 3 fois avec 10 mM de tris-HCl, pH 7,2, 150 mM de NaCl, 1% de triton X-100, 0,1% de SDS, 1% de sodium de desoxycholate et 2 fois avec 125 mM de Tris-Hcl, pH 6,8.

Enfin, les protéines ont été éluées en faisant bouillir la sépharose dans 40 µl d'une solution tampon pour électrophorèse sur gel.

L'électrophorèse sur gel de 15% de polyacrylamide a été réalisée selon la méthode de Laemli.

Après traitement par fluorographie, le gel a été séché et exposé à un film Kodak XAR-5 à −70°C

On constate ainsi que les particules d'HBsAg sont très résistantes à la trypsine dans des conditions non réductrices alors que l'HBsPolioAg est complètement clivé à un site unique (ou plusieurs sites proches) en produisant des polypeptides de poids moléculaires apparents de 17,4 et 14,3 kDa.

Les tailles des fragments sont compatibles avec le clivage de la séquence insérée.

En présence d'un agent réducteur, l'HBsAg est clivé exclusivement au niveau de l'Arg-122 (Peterson, D.L.), en générant des fragments de 16,6 et 13,2 kDa alors que l'HBsPolioAg est clivé en deux fragments de 17,4 et 13,2 kDa.

Ceci indique que le fragment de 14,3 kDa obtenu dans des conditions non réductrices à partir d'HBsPolioAg contient Arg 122 et est plus long de 10-12 amino-acides au niveau N-terminal que le fragment de 13,2 kDa, et confirme que le clivage de particules d'HBsPolioAg dans des conditions non réductrices s'est produit au niveau d'un ou des résidus lys de la séquence insérée (figure 2).

Ces résultats démontrent que la séquence peptidique insérée est facilement accesible aux protéases, c'est-à-dire qu'elle est exposée à la surface des particules hybrides d'enveloppe. Chez le virus polio du type 1 (Mahoney), la séquence peptidique correspondante a une structure moins exposée qui rend le résidu lys inaccessible à la trypsine dans des conditions réductrices (Fricks et al).

Les autres sites de clivage potentiels dans les particules hybrides d'HBsPolioAg sont inaccessibles pour la trypsine indiquant ainsi que ces parties de la molécule d'HBsAg reste dans une organisation très structurée.

Quelques changements conformationnels toutefois peuvent avoir lieu dans la région antigénique majeure HBs.

Ceci a été indiqué par la réduction (environ 20 fois) de la liaison d'anticorps anti-HBsAg aux particules d'HBsAg déterminée par radioimmunologie, en utilisant des particules d'HBsAg comme référence et détermination des protéines par coloration à l'argent après SDS-PAGE.

Des expériences d'immunoprécipitation d'HBsAg et d'HBsPolioAg par différents sérums indiquent que les deux particules réagissent avec des anticorps anti-HBsAg, et que les particules d'HBsPolioAg sont spécifiquement immunoprécipitées par les anticorps monoclonaux $C_3$ neutralisant le poliovirus, et par deux antisérums différents contre des oligopeptides synthétiques qui contiennent la séquence insérée.

Afin d'évaluer les propriétés immunogéniques des particules hybrides, des souris ont été immunisées avec HBsAg ou HBsPolioAg (Tableau 1), des ascites ont été créées intrapéritonéalement au moyen de cellules tumorigènes ne produisant pas d'anticorps afin d'obtenir un fluide ascitique immunologiquement similaire au sérum de souris (Anacker et al).

La vaccination avec HBsAg conduit à un haut titre en anticorps réagissant avec des particules humaines d'HBsAg (souris n° 1).

Toutefois, les souris immunisées avec l'HBsPolioAg répondent seulement faiblement aux antigènes HBs (souris n° 2 et 4, tableau Ib).

Ceci est en accord avec l'observation du fait que les déterminants antigéniques HBs sont partiellement distordus dans les particules hybrides.

La séquence insérée de poliovirus VP1, d'autre part, était immunologiquement active et induit, chez toutes les souris, des anticorps reconnaissant les peptides synthétiques porteurs de cette séquence (tableau Ic) ainsi que la protéine VP1 entière du poliovirus type 1 (Western blot). De plus, les antisérums obtenus possèdent une efficacité spécifique pour les virus infectieux ainsi que pour ceux dénaturés par la chaleur comme l'indiquent les expériences d'immunoprécipitation du tableau 1d. Plus encore, tous les antisérums possèdent un titre significatif en anticorps neutralisant les poliovirus (tableau le).

Des résultats préliminaires ont montré que les particules d'HBsAg sont également immunogéniques chez les lapins : après injection de deux doses d'HBspolioAg (10-40 µg chacune) 3 animaux sur 4 possèdent des anticorps immunoprécipitant avec les poliovirions infectieux.

Le potentiel que possèdent les particules HBsPolioAg d'entraîner l'apparition d'anticorps neutralisant reconnaissant un épitope rare commun aux poliovirions infectieux et dénaturés par la chaleur (Emini et al) montre que l'activité de la liaison aux anticorps et d'immunogénicité de la séquence aminoacide correspondante est exprimée au moins en partie à la surface des particules d'enveloppe HBV.

Cette courte séquence forme un pic sur la capside du poliovirus (Hogle et al) et peut par conséquent avoir une structure autonome.

Dans les particules d'HBsPolioAg, les séquences avoisinantes de l'HBsAg pourraient en plus maintenir la stabilité ou la flexibilité de la séquence de poliovirus inséré.

TABLEAU I

| SOURIS N° | ANTICORPS DE SOURIS CONTRE | TITRE EN ANTI-HBsAg (I.U) | RECONNAISSANCE PEPTIDE SYNTHETIQUE | ACTIVITE ANTIPOLIOVIRUS IMMUNOPRECIPITATION (%) | | TITRE: NEUTRALISANT (log 2) |
|---|---|---|---|---|---|---|
| | | | | VIRIONS INFECTIEUX | VIRIONS DENATURES | |
| | (a) | (b) | (c) | (d) | | (e) |
| 1 | HBsAg | 100 | − | 3 | 3 | 0 |
| 2 | HBsPolioAg | 0.01 | + | 89 | 96 | 4.36 |
| 3 | HBsPolioAg | 0 | + | 71 | 35 | 2.87 |
| 4 | HBsPolioAg | 0.1 | + | 90 | 87 | 3.89 |
| 5 | PLACEBO | 0 | − | 3 | 2 | 0 |

Les études des propriétés immunogèniques de l'HBsAg et de l'HBsPolioAg, correspondant aux résultats précédemment commentés du tableau I, ont été réalisées de la manière suivante :

a) Des souris Balb/C de 8 semaines ont été immunisées avec soit 2 µg d'HBsAg (n° 1), soit 30 µg d'HBsPolioAg (n° 2-4), purifiés comme il l'a été précédemment décrit, par injection intrapéritonéale en association avec une émulsion à 50% d'adjuvant complet de Freund, renouvelée deux semaines plus tard avec de l'adjuvant incomplet de Freund.

Après trois semaines, des cellules de myélome de souris sp2/0-Ag14 (Couillin et al) ont été injectées, suivi par une injection de rappel sans adjuvant.

La souris n° 5 a été traitée sans antigène. Les fluides ascitiques ont été collectés après deux semaines et ont été analysés suivant les procédés qui suivent.

b) Les titres en anti-HBsAg ont été déterminés par le test radioimmunologique AU5AB (Abott) et sont exprimés en unités internationales (I.U.)

c) La liaison au peptide correspondant aux aminoacides 93-104 des poliovirus VP1 a été déterminé par la méthode ELISA (Voller et al).

Les puits ont été enduits avec ce peptide (0,5 µg dans le PBS) pendant une nuit, et les sites inoccupés ont été bloqués avec du BSA (1% dans le PBS contenant 0,05% de tween-20).

Après lavages répétés avec du Tween-20 0,05% dans le PBS, les fluides ascitiques ont été additionnés à une dilution de 1 : 80 dans du PBS contenant 1% de BSA et 0,05% de Tween-20 et ont été incubés pendant 2 heures à 37°C.

Les puits ont été lavés, et des anticorps IgG anti-souris de chèvre (cappell) marqués à la peroxydase et dilués au 1/1000 ont été additionnés.

Après lavage, addition d'o-phenylènediamine (Merck : 0,5 µg/ml) dans 50 mM de tampon citrate/phosphate, pH 5, puis après 10 mn à température ambiante, la réaction est arrêtée par addition d'$H_2SO_4$ 2,5%.

Les sérums positifs sont ceux présentant une valeur d'absorption (D0) au moins trois fois supérieure à celle du contrôle (souris n° 5).

d) Le poliovirus type 1 (Mahoney) a été marqué avec $^{35}$S-Met et purifié par centrifugation dans un gradient de CsCl.

Les virions dénaturés par la chaleur ont été préparés par incubation des virions infectieux pendant une heure à 56°C.

Des aliquots de 50 µl contenant 15 000 cpm de particules marqués au $^{35}$S-Met (Emini et al) en solution dans 150 mM de NaCl, 5 mM d'EDTA, 50 mM de tris, pH 7,4, 0,02% de $NaN_3$ et 0,05% de Nonid et P40, ont été incubés en présence de 50 µl de fluides d'ascites de souris pendant une heure à 37°C et pendant une nuit à 4°C.

Les complexes immuns ont été précipités par <u>Staphilococcus aureus</u> (souche Cowan I) (Kessler) et leur radioactivité a été testée. Les chiffres du tableau Id représentent, en pourcentage, les valeurs de radioactivité immunoprécipitée.

e) Le titre en anticorps neutralisant de chaque sérum a été mesuré par test de réduction sur plaque standard avec des cellules Vero, en ajoutant une quantité de cent unités formant plaque (plaque forming units) de virus polio type 1 (Mahoney).

Les dilutions inverses de sérums (Log 2) donnant 5% de réduction sur plaque ont été calculées à partir des courbes de régression des valeurs moyennes obtenues à partir de 3 expériences.

Les expériences avec le gène modifié du plasmide pPAP montrent que les séquences étrangères insérées dans la protéine peuvent se trouver exposées à la surface des particules. Si la conformation initiale des séquences exogénes est modifiée, il est raisonnable de penser que l'insertion de structures plus grandes ou la delétion de certaines parties de la protéine HBsAg peuvent résoudre ce problème. La recherche d'autres sites d'insertion est aussi possible. Une insertion de 8 acides aminés au niveau du residu 50 de l'HBsAg (au site Bal I du gène S) permet la production de particules et leur excrétion.

L'invention permet par conséquent la production de vaccins mixtes.

Si l'on insère la séquence de déterminants antigéniques étrangers à ceux de l'HBsAg, il devient possible, si ces derniers son exposés en surface, de fabriquer des vaccins mixtes contre un autre sous-type du virus HBV, contre de déterminants du core du virus (HBcAg, HBeAg) (A.M. Prince et al. 1983, S. Iwarson et AL. 1984) contre les déterminants antigéniques de virus touchant les mêmes populations que l'hépatite B (SIDA, Herpès) et contre les déterminants antigéniques d'autres virus (T.M. Shinnick et al. 1983). Les huit acides aminés intervenant dans le déterminant antigénique majeur de la protéine $VP_1$ du poliovirus type 3 (D.M.A. (Evans et al. 1983) peuvent être insérés dans la protéine HBsAg par l'intermédiaire d'un fragment d'ADN synthétisé par voie chimique.

Ce type de manipulation peut aussi permettre l'expression de séquences biologiquement actives ayant un

EP 0 201 416 B1

intérêt pharmaceutique.

Les particules produites par les Hépadna virus animaux (P.L. Marion et al. 1983) peuvent être utilisées dans les mêmes conditions.

A ce titre, l'invention concerne toute composition de vaccin contre l'hépatite virale B contenant une dose efficace de particules conformes à l'invention, notamment de 3 à 6 microgrammes de protéine/ml, par exemple 5 microgrammes de protéine/ml (dose unitaire), en association avec un véhicule pharmaceutique approprié au mode d'administration choisi, notamment par voie parentérale.

Les pages qui suivent renvoient à la documentation antérieure à laquelle il a été fait référence dans ce qui précède.

## REFERENCES

– Anacker, R.L., Munoz, J.J. Immunol. 87, 426-432 (1961).

– Cattaneo, R., Will, H., Hernandez, N., Schaller, H. (1983) Nature, 305, 336-338.

– Colbère-Garapin, F., Horodniceanu, F., Kourilsky, P., Garapin, A.C. (1981) J. Mol. Biol., 150, 1-14.

– Couillin, P., Crainic, R., Cabau, N., Horodniceanu, F. & Boué, A. Ann Virol. (Inst. Pasteur) 133E, 315-323 (1982).

– Dubois, M.F., Pourcel, C., Rousset, S., Chany, C. et Tiollais, P. (1980) Proc. Natl. Acad. Sci. USA 77, 4549-4553.

– Emini, E.A., Bradford, A.J., Wimmer, E. Nature 304, 699-703 (1983).

– Evans, D.M.A., Minor, P.D., Schild, G.S., Almond, J.W. (1983) Nature 304, 460-462.

– Fricks, C.E., Icenogle, J.P., Hogle, J.M., J. Virol. 54, 856-859 (1985).

– Graham, F.L., Van der Eb, A.J. (1973) Virology, 52, 456.

– Gross-Bellard, M., Oudet, P., Chambon, P. (1973) Europ. J. Biochem. 36, 32.

– Hogle J.M., Chow, M., Filman, D.J. Science 229, 1358-1365 (1985).

– Hollinger, F.B., Sanchez, Y., Troisi, C., Dressman, G.R. Melnick, J.L. (1984) The 1984 International Symposium on Viral Hepatitis San Francisco, USA.

– Iwarson, S., Tabor, E., Thomas, H., Snoy, P., Gerety, R.J. (1984) The 1984 International Symposium on Hepatitis Virus, San Francisco, USA.

– Kaczorek, M., Delpeyroux, F., Chenciner, N., Streeck, R.E., Murphy, J.R., Boquet, P., Tiollais, P. (1983), Science 221, 853-855.

– Kessler, S.W. J. Immunol. 115, 1617-1624 (1975).

– Laemmli, U.K. (1970) Nature, 227, 680-685.

– Lusky, M., Botchan, M. (1981) Nature, 293, 79.

– Machida, A., Kishimoto, S., Ohnuma, H., Miyamoto, I., Baba, K., Oda, K., Nakamura, T., Funatsu, G., Mijakawa, Y., Mayami, M. (1982) Mol. Immunol. 19, 1087-1093.

– Marion, P.L., Knight, S.S., Feitelson, M.A., Oskiro, L.S., Robinson, W.S. (1983) Journal of Virology, 48, 534-541

– Maxam, A., Gilbert, W. (1980), Methods Enzymol. 65, 499.

– Michel, M.L., Pontisso, P., Sobczak, E., Malpiece, Y., Streek, R.E., Tiollais, P. (1984), Proc. Natl. Acad. Sci. USA 81, 7708-7712.

– Moriarty, A.M., Hoyer, B.H., Wai-Kuo Shih, J., Gerin, J.L., Hamer, D.H. (1981) Proc. Natl. Acad. Sci. USA 78.

– Neurath, A.R., Kent, S.B.H., Strick, N. (1984) The 1984 International Symposium on Hepatitis Virus, San Francisco, USA.

– Newmark, P. (1984), Nature 311, 510-511

– Pasek, M. et al, Nature (London) 282-575 (1979).

– Peterson, D.L., Paul, D.A., Gavilanes, F., Achord, D.T., in : Advances in Hepatitis research (ed. Chisari, F.V.) 30-39 (Mason Publishing U.S.A, Inc. 1984).

– Pillot, J., Petit, M.A. (1984) Molecular Immunology, 21, 53-60.

– Prince, A.M., Vnek, J., Stephan, W. (1983) Develop. Biol. Standard. 54, 13-22 (S. Kargel, Basel).

– Rigby, P.W.J., Dieckmann, M., Rhodes, C., Berg, P. (1977) J. Mol. Biol. 113, 237.

– Shapiro, S.K., Chou, J., Richaud, F.V. Casadaban, M.J. (1983) Gene, 25, 71-82.

– Smith, G.L., Mackett, M., Moss, B. (1983) Nature, 302, 490-495.

– Southern, E.M. (1975) J. Mol. Biol. 98, 503-517.

– Stibbe, W., Gerlich, W. (1983) Journal of Virology 46, 626-628.

– Tiollais, P., Charnay, P., Vyas, G.N. ((1981) Science 212, 406-411.

13

– Valenzuela, P., Medina, A., Rutter, W.J. (1982) Nature, 298, 347-350.

– Valenzuela, P. In Proceedings of the Twelth International Conference on Yeast Genetics and Molecular Biology. Edinburgh 1984, 16.

– Van der Werf, S., Wychowski, C., Bruneau, P., Blondel, B., Crainic, P.. Horodniceanu, F., Girard, M. (1983),Proc. Natl. Acad. Sci. USA 80, 5080-5084.

– Voller, A., Bedwell, D.E. & Berlett, A. A Guide with Abstracts of Microplate Applications, p. 1 (Dynatech, Guernesey 1979).

– Wigler, H., Pellicer, A., Silverstein, S., Axel, R., Urlaub G., Chasin, L. Proc. Natl. Acad. Sci. U.S.A 76, 1373-1376 (1979).

## Revendications

1. Particules contenant une proportion suffisante des séquences d'aminoacides caractéristiques du polypeptide majeur de l'antigène HBs du virus de l'hépatite virale B, pour conserver à ces particules une conformation similaire à celle des particules naturelles, caractéristique des antigènes d'enveloppe du virus de l'hépatite B, caractérisées en ce qu'elles comportent à leur surface une séquence d'acides aminés étrangère, de préférence porteuse d'un site immunogène, ladite séquence étrangère étant incorporée à l'intérieur même de ce polypeptide majeur en l'une de ses régions hydrophiles normalement exposées à la surface extérieure desdites particules ou, en variante, ladite séquence étrangère étant substituée à un ou plusieurs aminoacides appartenant à ces régions hydrophiles.

2. Particules selon la revendication 1, caractérisées en ce que la séquence étrangère d'aminoacides est insérée dans la région s'étendant entre les acides aminés 32 et 74 du polypeptide majeur.

3. Particules selon la revendication 1, caractérisées en ce que la séquence étrangère d'aminoacides est insérée dans la région s'étendant entre les acides aminés 110 et 156 du polypeptide majeur.

4. Particules selon l'une quelconque des revendications 1 à 3, caractérisées en ce que la séquence polypeptidique étrangère a une taille ne dépassant pas 16 aminoacides, notamment une taille de 5 à 16, ou même de 6 à 13, aminoacides.

5. Particules selon l'une quelconque des revendications 1 à 4, caractérisées en ce qu'elles contiennent également la séquence polypeptidique codée par la région pré-S du génome du virus de l'hépatite virale B.

6. ADN recombinant contenant une séquence d'ADN codant pour la région S et, le cas échéant, pré-S du polypeptide majeur du virus de l'hépatite virale B, caractérisé en ce qu'il comprend au moins une séquence nucléotidique codant pour une séquence d'aminoacides étrangère, ladite séquence nucléotidique étant au niveau de l'une au moins de celles des zones de la région S correspondant aux régions hydrophiles du polypeptide majeur du virus de l'hépatite B susdit, et en ce que la région S, et le cas échéant, la région pré-S, présentes dans l'ADN recombinant, sont placées sous le contrôle direct d'un promoteur exogène dont est connue la capacité de permettre l'initiation efficace de la transcription des gènes directement sous son contrôle dans les cellules eucaryotes, notamment humaines ou animales, ou encore dans les levures auxquelles lesdits vecteurs sont destinés.

7. ADN recombinant selon la revendication 6, caractérisé en ce que la séquence nucléotidique codant pour la susdite séquence étrangère est localisée dans la région du gène S qui code pour le polypeptide qui s'étend entre les acides aminés 32 et 74 du polypeptide majeur entrant dans la constitution de l'antigène HBs.

8. ADN recombinant selon la revendication 6, caractérisé en ce que la séquence nucléotidique codant pour la susdite séquence étrangère est localisée dans la région du gène S qui code pour le polypeptide qui s'étend entre les acides aminés 110 et 156 du polypeptide majeur entrant dans la constitution de l'antigène HBs.

9. ADN recombinant selon l'une quelconque des revendications 6 à 8, caractérisé en ce que la susdite séquence nucléotidique étrangère code pour un polypeptide ne dépassant pas 16 aminoacides et plus particulièrement de 6 à 13 aminoacides.

10. ADN recombinant selon l'une quelconque des revendications 6 à 9, caractérisé en ce que le susdit promoteur exogène est constitué par l'un des promoteurs du virus SV40.

11. Lignée cellulaire provenant d'un hôte, notamment humain ou animal, ou encore de levure capable de reconnaître le promoteur exogène de l'ADN recombinant selon l'une quelconque des revendications 6 à 10 caractérisée en ce qu'elle comporte, incorporé dans le génome, un ADN recombinant conforme à l'une quelconque des revendications 6 à 10.

12. Lignée cellulaire selon la revendication 11, caractérisée en ce qu'elle est formée de cellules reconnaissant le promoteur du virus SV40.

13. Procédé de production de particules selon l'une quelconque des revendications 1 à 5, caractérisé par la transformation d'une lignée cellulaire, notamment humaine ou animale, par un ADN recombinant conforme à l'une quelconque des revendications 6 à 10, le promoteur de l'ADN recombinant étant choisi en conformité avec la nature de l'hôte cellulaire utilisé, en ce que l'on cultive le micro-organisme ainsi transformé et en ce que l'on recueille les particules produites.

14. Procédé selon la revendication 13, caractérisé en ce que la séquence étrangère incorporée dans le polypeptide majeur comprend au plus 16 aminoacides et en ce que l'on récupère les particules produites excrétées dans le milieu de culture du susdit hôte cellulaire.

## Ansprüche

1. Teilchen entahltend einen Anteil von charakteristischen Aminosäuresequenzen des Hauptpolypeptids des HBs-Antigens des Virus von Virushepatitis B, der dazu ausreicht, eine Konformation dieser Teilchen aufrechtzuerhalten, die ähnlich ist derjenigen der natürlichen Teilchen und charakteristisch für die Antigene des Hauptpolypeptids der Hülle des Virus **dadurch gekennzeichnet**, daß sie auf ihrer Oberfläche eine fremde Aminosäuresequenz aufweisen, die vorzugsweise einen Immunogenort trägt, welche Fremdsequenz in das eigentliche Innere dieses Hauptpolypeptids in einen seiner hydrophilen Bereiche, die normalerweise auf der Obläche der Teilchen freiliegen, eingeführt ist, oder welche Fremdsequenz mit einer oder mehreren Aminosäuren substituiert ist, die diesen hydrophilen Bereichen angehören.

2. Teilchen nach Anspruch 1, **dadurch gekennzeichnet**, daß die fremde Aminosäuresequenz in den Bereich eingefügt ist, der sich zwischen den Aminosäuren 32 und 74 des Hauptpolypeptids erstreckt.

3. Teilchen nach Anspruch 1, **dadurch gekennzeichnet**, daß die fremde Amonosäuresequenz in den Bereich eingefügt ist, der sich zwischen den Aminosäuren 110 und 156 des Hauptpolypeptids erstreckt.

4. Teilchen nach einem der Ansprüche 1 bis 3, **dadurch gekennzeichnet**, daß die fremde Polypeptidsequenz eine Größe von nicht mehr als 16 Aminosäuren, namentlich eine Größe von 5 bis 16 oder bevorzugter von 6 bis 13 Aminosäuren aufweist.

5. Teilchen nach einem der Ansprüche 1 bis 4, **dadurch gekennzeichnet**, daß sie zusätzlich die Polypeptidsequenz enthalten, die durch den Prä-S-Bereich des Genoms des Virus der Virushepatitis B codiert ist.

6. Rekombinante DNA enthaltend eine DNA-Sequenz, die für den Bereich S und gegebenenfalls den Bereich Prä-S des Hauptpolypeptids des Virus der Virushepatitis B codiert, **dadurch gekennzeichnet**, daß sie mindestens eine Nucleotidsequenz enthält, die für eine fremde Aminosäuresequenz codiert, welche Nucleotidsequenz im Bereich mindestens einer der Zonen des Bereichs S entsprechend den hydrophilen Bereichen des oben angesprochenen Hauptpolypeptids des Virus von Hepatitis B angeordnet ist und daß der Bereich S und gegebenenfalls der Bereich Prä-S, die in der rekombinanten DNA vorhanden sind, unter der direkten Kontrolle eines exogenen Promotors stehen, von dem bekannt ist, daß er die Fähigkeit besitzt, eine wirksame Initiierung der Transcription von Genen direkt unter seinem Einfluß in eukariotischen Zellen, insbesondere menschlichen oder tierischen Zellen, oder auch in Hefen, für die die Vektoren bestimmt sind, zu initiieren.

7. Rekombinante DNA nach Anspruch 6, **dadurch gekennzeichnet**, daß die für die genannte Fremdsequenz codierende Nucleotidsequenz in dem Bereich des Gens S lokalisiert ist, der für das Polypeptid codiert, welches sich zwischen den Aminosäuren 32 und 74 des an dem Aufbau des Antigens HBs beteiligten Hauptpolypeptids erstreckt.

8. Rekombinante DNA nach Anspruch 6, **dadurch gekennzeichnet**, daß die für die genannte Fremdsequenz codierende Nucleotidsequenz in dem Bereich des Gens S lokalisiert ist, der für das Polypeptid codiert, welches sich zwischen den Aminosäuren 110 und 156 des an dem Aufbau des Antigens HBs beteiligten Hauptpolypeptids erstreckt.

9. Rekombinante DNA nach einem der Ansprüche 6 bis 8, **dadurch gekennzeichnet**, daß die genannte fremde Nucleotidsequenz für ein Polypeptid codiert, welches 16 Aminosäuren und insbesondere 6 bis 13 Aminosäuren nicht übersteigt.

10. Rekombinante DNA nach einem der Ansprüche 6 bis 9, **dadurch gekennzeichnet**, daß der exogene Promotor einer der Promotoren des Virus SV40 ist.

11. Aus einem namentlich menschlichen oder tierischen Wirt stammende Zellinie oder Hefe, die in der Lage ist, den exogenen Promotor der rekombinanten DNA nach einem der Ansprüche 6 bis 10 zu erkennen, **dadurch gekennzeichnet**, daß sie in das Genom inkorporiert eine rekombinante DNA nach einem der Ansprüche 6 bis 10 enthält.

12. Zellinie nach Anspruch 11, **dadurch gekennzeichnet**, daß sie aus Zellen gebildet ist die den Promotor des Virus SV40 erkennen.

13. Verfahren zur Herstellung der Teilchen nach einem der Ansprüche 1 bis 4, **dadurch gekennzeichnet**, daß man eine insbesondere menschliche oder tierische Zellinie mit einer rekombinanten DNA nach einem der Ansprüche 6 bis 10 transformiert, wobei der Promotor der rekombinanten DNA in Abhängigkeit von der Art des verwendeten Zellwirts ausgewählt wird, und man den in dieser Weise transformierten Mikroorganismus züchtet und die gebildeten Teilchen gewinnt.

14. Verfahren nach Anspruch 13, **dadurch gekennzeichnet,** daß die in das Hauptpolypeptid eingeführte Fremdsequenz höchstens 16 Aminosäuren enthält und man die gebildeten Teilchen gewinnt, welche von dem genannten Zellwirt in dem Kulturmedium ausgeschieden worden sind.

## Claims

1. Particles containing a sufficient proportion of the sequences of amino acids characteristic of the major polypeptide of the HBs antigen of the viral hepatitis B virus, for these particles to retain a structure similar to the structure of the natural particles characteristic of the enveloppe antigens of the hepatitis B virus characterized in that they comprise at their surface, by the incorporation into this major polypeptide of a foreign sequence of amino acids, preferably itself carrier of an immunogenic site, said sequence been incorporated within this major polypeptide, in one of its hydrophilic regions normally exposed at the external surface of the said particles, or as an alternative, by the substitution of one or more amino acids forming part of these hydrophilic regions by the said foreign sequence of amino acids.

2. Particles according Claim 1, characterized in that the foreign sequence of amino acids is inserted in the region extending between amino acids 32 and 74 of the major polypeptide.

3. Particles according to Claim 1, characterized in that the foreign sequence of amino acids is inserted in the region extending between the amino acids 110 and 156 of the major polypeptide.

4. Particles according to any one of the Claims 1 to 3, characterized in that the foreign polypeptide sequence has a size not exceeding 16 amino acids, in particular a size of from 5 to 16, or even from 6 to 13 amino acids.

5. Particles according to any one of Claims 1 to 4, characterized in that they also contain the polypeptide sequence encoded in the pre-S region of the genome of the hepatitis B virus.

6. Recombinant DNA containing a DNA sequence coding for the S region, and if appropriate, the pre-S region of the genome of the major polypeptide of the hepatitis B virus, characterized in that it comprises at least one nucleotide sequence coding for the above-mentioned foreign amino acids sequence said sequence being at the level of at least one of those zones of the S region corresponding to the hydrophilic regions of the above-mentioned major polypeptide of the hepatitis B virus, and in that the S region and, if appropriate, the pre-S region, present in the recombinant DNA, are placed under the direct control of an exogenous promoter with the known capacity for allowing efficient initiation of the transcription of the genes directly under its control in the eucaryotic cells, in particular human or animal cells, or even yeasts, for which the said vectors are intended.

7. Recombinant DNA according to Claim 6, characterized in that the nucleotide sequence coding for the above-mentioned foreign sequence is localized in the region of the S gene which codes for the polypeptide which extends between the amino acids 32 and 74 of the major polypeptide forming part of the structure of the HBs antigen.

8. Recombinant DNA according to Claim 6, characterized in that the nucleotide sequence coding for the above-mentioned foreign sequence is localized in the region of the S gene which codes for the polypeptide which extends between the amino acids 110 and 156 of the major polypeptide forming part of the structure of the HBs antigen.

9. Recombinant DNA according to any one of the Claims 6 to 8, characterized in that the above-mentioned foreign nucleotide sequence codes for a polypeptide not exceeding 16 amino acids and more particularly of from 6 to 13 amino acids.

10. Recombinant DNA according to any one of the Claims 6 to 9, characterized in that the above-mentioned exogenous promoter is constituted by one of the promoters of the SV40 virus.

11. Cell line derived from a host, in particular a human or animal host, or even a yeast capable of recognizing the exogenous promoter of the recombinant DNA according to any one of the Claims 6 to 10, characterized in that it contains, incorporated in the genome, a recombinant DNA in conformity with any one of the claims 6 to 10.

12. Cell line according to Claim 11, characterized in that it is composed of cells recognizing the promoter of the SV40 virus.

13. Procedure for the production of particles according to any one of the Claims 1 to 5, characterized by the transformation of the cell line, in particular a human or animal cell line, by a recombinant DNA in conformity with any one of the Claims 6 to 10, the promoter of the recombinant DNA being chosen in conformity with the nature of the cell host used, in that the microorganism thus transformed is cultivated and in that the particles produced are recovered.

14. Procedure according to Claim 13, characterized in that the foreign sequence incorporated into the major polypeptide comprises at most 16 amino acids and in that the excreted particles produced are recovered from the culture medium of the above-mentioned cell host.

# FIG.1

# FIG. 2

pML2

pPAP

SV40

HBV

BamH1
GGA TCC  GAT  AAC  CCA  GCG  TCG  ACC  ACG  AAT  AAG  GAT  AAG
GLY  SER  ASP  ASN  PRO  ALA  SER  THR  THR  ASN  LYS  ASP  LYS

BamH1
GGA TCC
GLY  SER

POLIOVIRUS